**Europäisches Patentamt**

**European Patent Office**

**Office européen des brevets**

(11) Veröffentlichungsnummer: **0 514 575 A1**

## (12) EUROPÄISCHE PATENTANMELDUNG

(21) Anmeldenummer: **91108406.9**

(22) Anmeldetag: **24.05.91**

(51) Int. Cl.⁵: **G01N 27/28**

(43) Veröffentlichungstag der Anmeldung:
**25.11.92 Patentblatt 92/48**

(84) Benannte Vertragsstaaten:
**AT BE CH DE DK ES FR GB IT LI LU NL SE**

(71) Anmelder: **Riemann, Werner**
**Heinrich-Heine-Strasse 11A**
**W-3550 Marburg/Lahn(DE)**

(72) Erfinder: **Riemann, Werner**
**Heinrich-Heine-Strasse 11A**
**W-3550 Marburg/Lahn(DE)**

(74) Vertreter: **Olbricht, Karl Heinrich, Dipl.-Phys.**
**Am Weinberg 15**
**W-3556 Niederweimar(DE)**

(54) **Analyse-Modul.**

(57) Zur Mikro-Analyse insbesondere von Vollblut, Serum o.dgl. enthält ein Modul (10) im Gehäuse (12) einen engen Meßkanal (14), an den eine Elektrode (16) reicht. Sie weist einen Schraubkopf (20) mit einem Anschluß (24) auf, der unter mechanischer Selbstzentrierung in einer Kontaktzone (30) mit einem zu einer Sensorfläche (48) führenden Ableitdraht (28) lösbar verbunden ist. Ein Zentrierrohr (68) hat eine Einlauföffnung (58) für einen ggf. verjüngten Mittenleiter (32) des Anschlusses (24), der in einem Sackloch (34) das freie Ableitdraht-Ende (36) leitend übergreift. Es trägt einen spanlose geformten Ringwulst (38). Der so verdickte, in einer Ringhalterung (66) des Elektrodenfußes (18) gefaßte Ableitdraht (28) verklemmt sich im bevorzugt mit Lot (60) ausgekleideten Sackloch (34) des Mittenleiters (32), der unten spreizbar sein kann. Ein Stützteil (50) schützt die Sensorfläche (48) im Fuß (18), der an einem Bund (46) mittels O-Ringen (44, 62) gegenüber Kopf (20) sowie Gehäuse (12) abgedichtet und durch eine Anschlagstufe (40, 42) reproduzierbar genau gehalten ist. Der Sensorfläche (48) steht im Meßkanal (14) eine symmetrische, von einem quer eingepaßten Pfropfen (56) gebildete Kuppe (54) gegenüber.

Fig. 1

Die vorliegende Erfindung betrifft ein Analyse-Modul gemäß dem Oberbegriff von Anspruch 1.

Derartige Einheiten sind beispielsweise in DE-U-8 705 520 beschrieben. Unter Einsatz ionenselektiver Elektroden verwendet man sie in der klinisch-chemischen Analytik. Dabei wird Meßflüssigkeit durch einen Meßkanal gefördert, an den zwei oder mehr hintereinander angeordnete Referenz- und Meßelektroden heranreichen, die als elektrochemische Halbzellen selektiv auf bestimmte Ionenarten ansprechen. Das bekannte Modul bedient sich einer Meßkammer in Form einer die Meßstrecke kreuzenden, beidseitig verschlossenen Bohrung, die einerseits von einer Elektrode, bevorzugt der Bezugselektrode, und andererseits von einem Verschlußelement flüssigkeitsdicht abgeschlossen ist, namentlich von einer semipermeablen Scheibe aus Silikonkautschuk. Dadurch können sehr kleine Luftmengen in die Kammer gelangen, die einen Unterdruck beim - extrem langsamen - Abfließen eines Elektrolyten verhindern. Bei Verwendung von Wegwerf-Pipettenspitzen lassen sich Meß- und Eichflüssigkeiten bequem und mit minimaler Verschleppungsgefahr in die Meßstrecke einbringen. Das Wechseln der empfindlichen Meßsysteme oder ein Übergang zu gassensitiven Elektroden ist jedoch problematisch und herkömmlich nicht oder nicht zuverlässig beherrscht. Häufig treten Schwierigkeiten aufgrund von Luftblasen auf, die im Meßkanal beharrlich hängenbleiben und nur schwer oder mit großem Durchströmvolumen beseitigbar sind.

Es besteht daher ein Bedürfnis an Abhilfe auf möglichst wirtschaftliche Art. Ein wichtiges Ziel der Erfindung ist es, unter Überwindung der Nachteile des Standes der Technik ein verbessertes Analyse-Modul zu entwickeln, das bei einfachem Aufbau vielfältige Anwendungen gestattet und auch in Arztpraxen, Kleinlabors und Kliniken rasch umgerüstet werden kann, ohne an Genauigkeit und Betriebszuverlässigkeit einzubüßen.

Hauptmerkmale der Erfindung sind im kennzeichnenden Teil von Anspruch 1 angegeben. Ausgestaltungen sind Gegenstand der Ansprüche 2 bis 20.

Bei einem Analyse-Modul mit einem z.B. aus Polymethylmethacrylat bestehenden Gehäuse, in dessen Meßkanal der Fuß einer Elektrode mit einer Sensorfläche heranreicht, von der ein Ableitdraht zu einem Anschluß an einem Schraubkopf der Elektrode führt, sieht die Erfindung vor, daß Ableitdraht und Anschluß unter mechanischer Zentrierung in einer Kontaktzone lösbar verbunden sind. Ein solches, überaus einfach aufgebautes Modul ist mit sehr kleinen Abmessungen realisierbar. Trotz der Feinheit der benutzten Elemente lassen sich die Bestandteile mühelos und exakt handhaben. Wenn sich eine Elektrode nach etlichen Wochen oder Monaten verbraucht hat, ist ein Austausch ohne weiteres möglich. Auch ein Wechsel zu einer anderen Elektrodenart ist problemlos möglich. Dabei kann es genügen, den Elektrodenfuß auszutauschen, während derselbe Kopf weiterverwendet wird. Aus- und Einbau vollziehen sich schnell und genau, so daß hohe Betriebszuverlässigkeit stets gewährleistet ist.

Dazu trägt es bei, wenn gemäß Anspruch 2 die Enden von Ableitdraht und Anschluß selbstzentrierend geführt, also Fehlmontagen ausgeschlossen sind. Laut Anspruch 3 kann der Elektrodenfuß ein Zentrierrohr aufweisen, welches das freie Ende des Ableitdrahtes in der Kontaktzone mit konzentrischem Abstand umgibt. Besonders günstig ist die Konstruktion von Anspruch 4, wonach das Zentrierrohr eine Einlauföffnung für einen Mittenleiter des Anschlusses aufweist oder bildet. Zusätzlich kann der Mittenleiter gemäß Anspruch 5 am unteren Ende zu einem Einlaufteil von Spitzbogen-Gestalt verjüngt sein. Diese Maßnahmen unterstützen die Schnelligkeit und Genauigkeit der Montage erheblich.

Nach Anspruch 6 weist der Mittenleiter am unteren Ende ein Sackloch auf, in dem das freie Ende des Ableitdrahtes mit leitender Verbindung festgelegt bzw. festlegbar ist. Dieses Sackloch stülpt sich mit der vorbeschriebenen Selbstzentrierung über den Ableitdraht, der laut Anspruch 7 nahe seinem freien Ende einen Ringwulst hat, welcher sich im Sackloch verklemmt. Dazu überschreitet er dessen lichte Weite wenigstens geringfügig, z.B. in einem Durchmesserverhältnis gemäß Anspruch 8, vorzugsweise um etwa 10 %. Wahlweise oder zusätzlich kann das untere Ende des Mittenleiters im Einklang mit Anspruch 9 an achsparallelen Schlitzen spreizbar sein.

Die leitende Verbindung zwischen Ableitdraht und Anschluß wird nach Anspruch 10 wesentlich dadurch unterstützt, daß in der Kontaktzone eine Lotschicht oder Teil-Lotfüllung vorhanden ist, die gemäß Anspruch 11 die Innenwandung des Sacklochs in solcher Stärke (z.B. 0,1 mm) überzieht, daß das freie Ende des Ableitdrahtes noch frei eintreten kann, sein Ringwulst jedoch zu sattem Formschluß mit dem Lot gelangt. Dieses wird beim Einschieben des Ableitdraht-Endes teilweise verdrängt, weshalb es zweckmäßig ist, in einer eventuell benutzten Lotfüllung einen genügenden Freiraum zu schaffen, etwa durch Aufbohren oder Räumen.

Herstellungsmäßig ist es günstig, den Ringwulst nach Anspruch 12 durch spanloses Drücken, Rollen o.dgl. am Ableitdraht zu formen. Dieser kann aus Reinsilber bestehen, an dem diese Bearbeitung besonders leicht durchführbar ist.

Nach Anspruch 13 ist der Ableitdraht in einer Ringhalterung gefaßt, bevorzugt unter Verklebung mittels Epoxidharz. Vorteilhaft ist dabei die Ringhalterung laut Anspruch 14 einstückig mit dem Elektrodenfuß, in dem der Ableitdraht nach Anspruch 15 einen Stützteil durchsetzen kann, an dessen Unterseite die Sensorfläche angeordnet ist. Der weiche und dünne Draht, dessen Durchmesser beispielsweise 0,5 mm bis 1,0 mm beträgt, ist auf diese Weise geschützt und zentrisch gehalten.

Zur mechanischen Stabilität trägt ferner die wichtige Maßnahme von Anspruch 16 bei, wonach der Ableitdraht unten einen Bund des Elektrodenfußes durchsetzt, der mittels O-Ringen einerseits gegenüber dem Kopf, andererseits gegenüber dem Gehäuse abgedichtet ist. Dies ist für den Betrieb außerordentlich wichtig, um Rückwirkungen durch die Meßflüssigkeit auszuschließen.

Gemäß Anspruch 17 kann der Kopf an seinem unteren Ende eine Schulter haben, die an einer Stufe im Gehäuse einen Anschlag findet, welche den exakten Sitz des Elektrodenfußes sicherstellt. Dazu trägt nach Anspruch 18 eine solche Bemessung des Bundes bei, daß dieser im Gehäuse satt passend geführt und ein Herausdrücken des Stützteils bzw. der Sensorfläche in den Meßkanal ausgeschlossen ist. Die ebenso kleinen wie empfindlichen Bauteile werden dadurch auf einfachste Weise geschützt.

Das Merkmal von Anspruch 19, wonach der Sensorfläche im Meßkanal eine Kuppe gegenübersteht, ist günstig für die Vermeidung von Luftblasen, die sich infolge der durch die Meßkanal-Verengung entstehenden Düsenwirkung gerade in der Meßzone nicht halten können. Mit besonders einfacher Fertigung läßt sich eine symmetrische Kuppe gemäß Anspruch 20 durch einen Propfen bilden, der quer zu dem Meßkanal in das Gehäuse eingepaßt wird und beispielsweise ein Rund- oder Ovalstab, ein Prismenkörper o.dgl. sein kann. Unabhängig von der Durchfluß-Richtung bleibt die Meßzone daher luftblasenfrei.

Weitere Merkmale, Einzelheiten und Vorteile der Erfindung ergeben sich aus dem Wortlaut der Ansprüche sowie aus der folgenden Beschreibung eines Ausführungsbeispiels anhand der Zeichnung. Darin zeigen:

Fig. 1     eine schematisierte Schnittansicht eines Analyse-Moduls,
Fig. 2     eine vergrößerte Darstellung ähnlich Fig. 1 und
Fig. 3     eine Schnittansicht der Einzelheit III von Fig. 2.

Das insgesamt mit 10 bezeichnete Analyse-Modul hat ein Gehäuse 12 mit einem Meßkanal 14, dessen Innendurchmesser z.B. 0,5 mm bis 1,0 mm beträgt. Von einer Gehäuseseite her reicht an den Meßkanal 14 eine Elektrode 16 mit einem Fuß 18 heran, der in einer Bohrung 19 des Gehäuses 12 sitzt. An einem Kopf 20 mit einem Gewindeteil 22 befindet sich ein Anschluß 24 für die Elektrode 16.

Erfindungsgemäß ist die Elektrode 16 auf besonders einfache Art ganz oder teilweise austauschbar. Dazu zerlegt man sie durch Abnahme des Kopfes 20, der sich mühelos aus- und einschrauben läßt, indem man ein (nicht gezeichnetes) Werkzeug in Ausnehmungen 26 steckt. Der freigelegte Fuß 18 kann von Hand oder mit Werkzeughilfe aus der Bohrung 19 gezogen bzw. in sie eingesetzt werden.

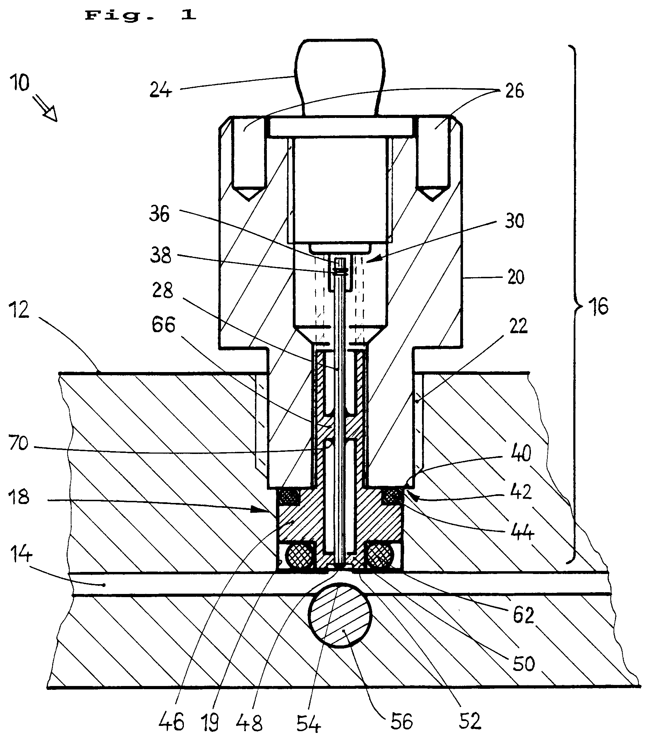

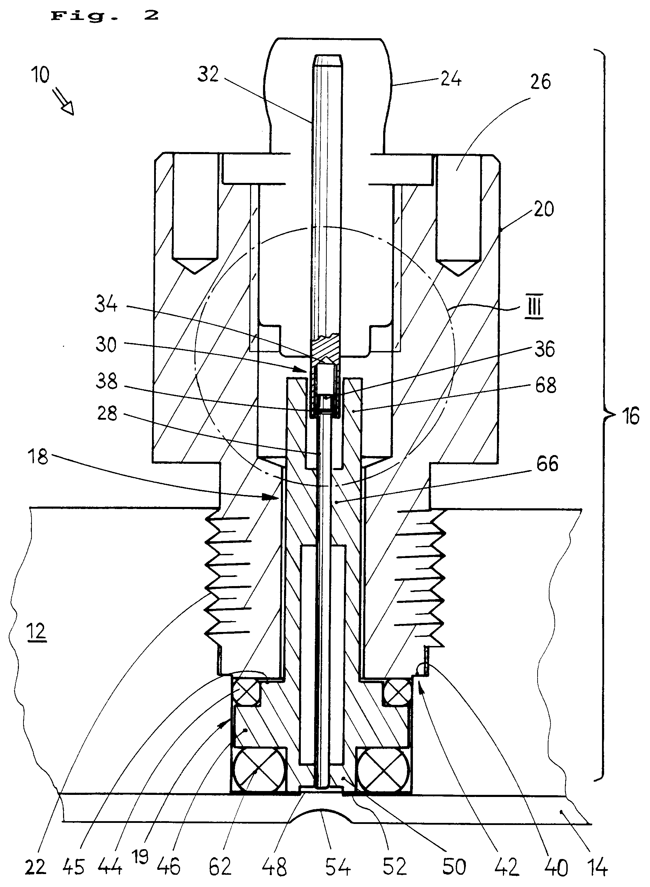

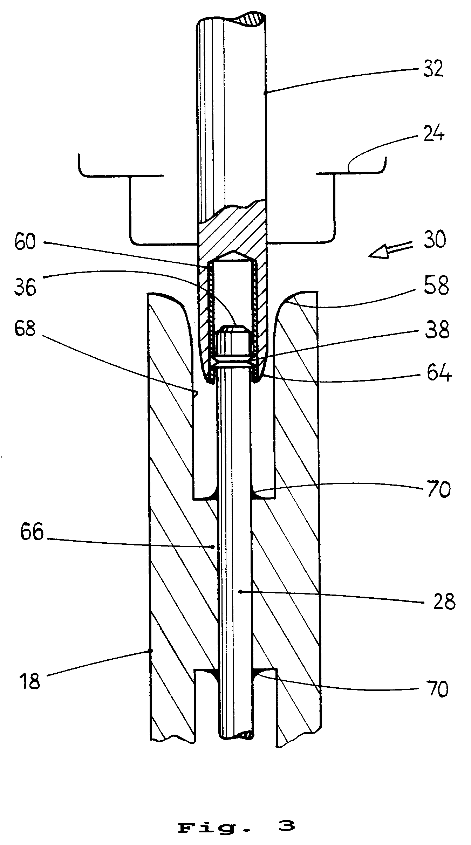

Sowohl bei der Montage als auch beim Ausbau ist eine selbstzentrierende Führung gewährleistet, durch die ein im Elektrodenfuß 18 gehalterter Ableitdraht 28 in einer Kontaktzone 30 die Verbindung mit einem Mittenleiter 32 des Anschlusses 24 herstellt bzw. löst. Einzelheiten sind aus Fig. 2 und 3 ersichtlich.

Der Mittenleiter 32 weist an seinem unteren Ende ein Sackloch 34 auf, mit dem er das freie Ende 36 des Ableitdrahtes 28 übergreift. Dieses ist mit einem Ringwulst 38 versehen, welcher sich bei der Montage im Sackloch 34 des Mittenleiters 32 verklemmt und dadurch die leitende Verbindung herstellt. Gegebenenfalls kann der Mittenleiter 32 unten mit (nicht gezeichneten) Schlitzen versehen sein, um ihn federnd spreizbar zu machen.

Der Kopf 20 hat am Gewindeteil 22 unten eine Schulter 40, die an einer Stufe 42 neben der Bohrung 19 im Block bzw. Gehäuse 12 einen Anschlag findet. Dadurch wird die Einschraubtiefe für den Kopf 20 begrenzt, um eine Beschädigung oder Fehlfunktion der Elektrode 16 auszuschließen.

Mit einem Bund 46 ist der Elektrodenfuß 18 in die Bohrung 19 satt einpaßbar. Daran schließt ein Stützteil 50 an, der eine Sensorfläche bzw. Membran 48 umfaßt, an die der Ableitdraht 28 geführt ist. Mit seiner Stirnfläche 52 reicht der Stützteil 50 an den Meßkanal 14 heran. Ein O-Ring 62 umgibt den Stützteil 50 zur Abdichtung des Bundes 46 nach unten. Zum Gewindeteil 22 des Kopfes 20 hin übernimmt die Abdichtung ein O-Ring 44, der zwar vom unteren Ende des Gewindeteils 22 in geringem Maße verformt werden kann, jedoch unter Beibehaltung eines kleinen Mindestabstandes zur Oberseite 45 des gestuften Bundes 46, weil die Schulter 40 des Kopfes 20 an der Stufe 42 des Gehäuses 12 anschlägt. Der erwähnte Mindestabstand kann beispielsweise 0,10 mm betragen.

Im Meßkanal 14 steht der Stirnfläche 52 des Stützteils 50 und mithin der Sensorfläche 48 eine Kuppe 54 (Fig. 2) gegenüber, die in einfacher Weise durch einen Pfropfen 56 gebildet ist, der als Rolle oder Prisma ausgebildet und quer zum Meßkanal 14 in eine vorbereitete Bohrung eingepreßt und/oder eingeklebt sein kann. Bevorzugt ist die Kuppe 54 symmetrisch ausgebildet, so daß unabhängig von der Durchflußrichtung im Meßkanal 14 etwa vorhandene Luftbläschen durch Düsenwirkung ausgeschwemmt werden.

Aus der weiter vergrößerten Schemazeichnung von Fig. 3 ist ersichtlich, daß die Kontaktzone 30 in besonderer Weise ausgestaltet sein kann, um eine leitende Verbindung zwischen dem Ableitdraht 28 der Elektrode 16 und dem Mittenleiter 32 des Anschlusses 24 rasch und zuverlässig herzustellen. Dazu kann am Zentrierrohr 68 eine Einlauföffnung 58 und am unteren Ende des Mittenleiters 32 ein verjüngter Einlaufteil 64 vorgesehen sein. Der Ableitdraht 28, der an einem Ringteil 66 des Elektrodenfußes 18 zweckmäßig mit Klebestellen 70 festgelegt ist, hat beispielsweise einen Durchmesser von 0,50 mm und ist am Ringwulst 38 auf 0,60 mm Durchmesser aufgeweitet. Hält man sich vor Augen, daß der Mittenleiter 32 mit z.B. 1,0 mm Außendurchmesser am Sackloch 34 nur eine geringe Wandstärke besitzen kann, so wird deutlich, wie wichtig die selbstzentrierende Führung in der Kontaktzone 30 ist. Wenn das Sackloch 34 eine lichte Weite von 0,70 mm hat und innen mit einer Lotschicht 60 derart ausgekleidet ist, daß die freie Weite immer noch 0,55 mm beträgt, so hat der Ringwulst 38 hierzu einen Überstand von nur 0,05 mm im Durchmesser. Das reicht allerdings völlig aus, um bei sicherer Einführung des freien Endes 36 zuverlässig eine leitende Verbindung über die Lotschicht 60 mit dem z.B. vergoldeten Mittenleiter 32 herzustellen. Auch wenn der Ableitdraht 28 aus Reinsilber besteht und (oberflächlich) chloriert ist, ergibt sich ein sicherer, gut leitender Kontakt.- Der Anschluß 24, welcher den Mittenleiter 32 metallisch faßt, kann als handelsüblicher Büschel-Kontakt ausgebildet sein.

Die Erfindung ist nicht auf die erläuterten Ausführungsformen beschränkt. Ein ionenselektiver Elektrodenkörper (ISE) hat an der Sensorfläche 48 gewöhnlich einen Polymerträger. Durch Auswechseln des Fußes 18 lassen sich gassensitive Elektroden einsetzen, die typisch aus Platindraht mit Elektrolyt und Membranabschluß bestehen. Form und Abmessungen können dabei den vorbeschriebenen exakt entsprechen, so daß ein und dieselben Elektrodenköpfe 20 mit unterschiedlich funktionierenden, aber gleichartig aufgebauten Elektrodenfüßen 18 verwendbar sind. In jedem Falle bleibt die einfache, betriebssichere Handhabung gewahrt.

Das erfindungsgemäße Modul 10 ist ohne weiteres zum Einsatz in einem Grundgerät beispielsweise nach GM 87 05 520 geeignet. Mit den Elektroden-Anschlüssen 24 läßt es sich bequem auf ein Meßgerät aufstecken, um in an sich bekannter Weise nach Nullabgleich Potentialdifferenzen messen zu können. Schon ein kleines Sensor-Sortiment kann bei Einsatz dieser Mikroanalytik unter Umständen ein Großlabor ersetzen. Für die Einzelproben-Analyse reicht eine Probenmenge von nur 5 μl bis 30 μl, die etwa durch Stich ins Ohrläppchen oder in die Fingerbeere entnommen werden kann. Das ist namentlich in der Säuglings-Medizin von großer Bedeutung.

Das erfindungsgemäße Analysen-Modul 10 ermöglicht einen mobilen, patientennahen Einsatz beispielsweise am Krankenbett, was mit großen Analysegeräten wegen ihrer Unhandlichkeit und ihres hohen Gewichts nicht möglich ist, und es liefert dem Arzt vor Ort einen klinisch relevanten Laborbefund, der ihm die Präsenzdiagnostik ermöglich. Infolgedessen kann er neben der Anamnese sofort eine Therapieentscheidung treffen. Das sehr kostengünstige Modul 10 eignet sich auch zur Verwendung fernab von externer Energieversorgung, was für den Einsatz in Entwicklungsländern von Bedeutung seinkann. Neben Schnellanalysen kommt auch die Verwendung für Forschungszwekke in Betracht. In Verbindung mit einem Grund-Meßblock ist ohne größeren Aufwand über ein Interface ein Anschluß an alle marktgängigen Mikrocomputer zur bequemen Auswertung möglich. Es lassen sich alle wichtigen Ionen wie z.B. $Na^+$, $K^+$, $Ca^{++}$, $Cl^-$ problemlos erfassen.

Man erkennt, daß nach der Erfindung zur Mikro-Analyse insbesondere von Vollblut, Serum o.dgl. ein Modul 10 im Gehäuse 12 einen engen Meßkanal 14 enthält, an den eine Elektrode 16 reicht. Sie weist einen Schraubkopf 20 mit einem Anschluß 24 auf, der unter mechanischer Selbstzentrierung in einer Kontaktzone 30 mit einem zu einer Sensorfläche 48 führenden Ableitdraht 28 lösbar verbunden ist. Ein Zentrierrohr 68 hat eine Einlauföffnung 58 für einen ggf. verjüngten Mittenleiter 32 des Anschlusses 24, der in einem Sackloch 34 das freie Ableitdraht-Ende 36 leitend übergreift. Es trägt einen spanlose geformten Ringwulst 38. Der so verdickte, in einer Ringhalterung 66 des Elektrodenfußes 18 gefaßte Ableitdraht 28 verklemmt sich im bevorzugt mit Lot 60 ausgekleideten Sackloch 34 des Mittenleiters 32, der unten spreizbar sein kann. Ein Stützteil 50 schützt die Sensorfläche 48 im Fuß 18, der an einem Bund 46 mittels O-Ringen 44, 62 gegenüber Kopf 20 sowie Gehäuse 12 abgedichtet und durch eine Anschlagstufe 40, 42 reproduzierbar genau gehalten ist. Der Sensorfläche 48 steht im Meßkanal 14 eine symmetrische, von einem quer eingepaßten Pfropfen 56 gebildete Kuppe 54 gegenüber.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

Legende PA 507 EP

| | |
|---|---|
| 10 | Analyse-Modul |
| 12 | Gehäuse / Meßblock |
| 14 | Meßkanal |
| 16 | Elektrode |
| 18 | Fuß |
| 19 | Bohrung |
| 20 | Kopf |
| 22 | Gewindeteil |
| 24 | Anschluß |
| 26 | Ausnehmungen |
| 28 | Ableitdraht |
| 30 | Kontaktzone |
| 32 | Mittenleiter |
| 34 | Sackloch |
| 36 | freies Ende |
| 38 | Ringwulst |
| 40 | Schulter |
| 42 | Anschlagstufe |
| 44 | O-Ring |
| 45 | Oberseite |
| 46 | Bund |
| 48 | Sensorfläche / Membran |
| 50 | Stützteil |
| 52 | Stirnfläche |
| 54 | Kuppe |
| 56 | Pfropfen |
| 58 | Einlauföffnung |
| 60 | Lotschicht, -füllung |
| 62 | O-Ring |
| 64 | Einlaufteil |
| 66 | Ring(teil) |
| 68 | Zentrierrohr |
| 70 | Klebestellen |

**Patentansprüche**

1. Modul (10) zur elektrochemischen Analyse von Flüssigkeiten wie Vollblut, Serum o.dgl., mit einem Gehäuse (12), das einen engen Meßkanal (14) enthält, an den der Fuß (18) einer quer dazu abgedichtet angeordneten Elektrode (16) heranreicht, die einen zu einer Sensorfläche (48) führenden Ableitdraht (28) aufweist, und mit einem Anschluß (24) an einem in das Gehäuse (12) einschraubbaren Kopf (20), dadurch **gekennzeichnet**, daß Ableitdraht (28) und Anschluß (24) unter mechanischer Zentrierung in einer Kontaktzone (30) lösbar verbunden sind.

2. Modul nach Anspruch 1, dadurch **gekennzeichnet**, daß die Enden von Ableitdraht (28) und Anschluß (24) selbstzentrierend geführt sind.

3. Modul nach Anspruch 1 oder 2, dadurch **gekennzeichnet**, daß der Fuß (18) der Elektrode (16) ein Zentrierrohr (58) aufweist, welches das freie Ende (36) des Ableitdrahtes (28) in der Kontaktzone (30) mit insbesondere konzentrischem Abstand umgibt.

4. Modul nach Anspruch 2 und 3, dadurch **gekennzeichnet**, daß das Zentrierrohr (68) eine Einlauföffnung (58) für einen Mittenleiter (32) des Anschlusses (24) aufweist oder bildet.

5. Modul nach Anspruch 4, dadurch **gekennzeichnet**, daß sich der Mittenleiter (32) am unteren Ende verjüngt, vorzugsweise zu einem Einlaufteil (64) in Form einer Spitzbogen-Rundung.

6. Modul nach Anspruch 4 oder 5, dadurch **gekennzeichnet**, daß der Mittenleiter (32) am unteren Ende ein Sackloch (34) aufweist, in dem das freie Ende (36) des Ableitdrahtes (28) mit leitender Verbindung festgelegt bzw. festlegbar ist.

7. Modul nach Anspruch 6, dadurch **gekennzeichnet**, daß der Ableitdraht (28) nahe seinem freien Ende (36) einen Ringwulst (38) aufweist, dessen Außendurchmesser die lichte Weite des Sacklochs (34) wenigstens geringfügig überschreitet.

8. Modul nach Anspruch 7, dadurch **gekennzeichnet**, daß sich der Außendurchmesser des Ringwulstes (38) zur lichten Weite des Sacklochs (34) wie 1,05 : 1 bis 1,20 : 1 verhält, vorzugsweise wie 1,10 : 1.

9. Modul nach einem der Ansprüche 4 bis 8, dadurch **gekennzeichnet**, daß das untere Ende des Mittenleiters (32) spreizbar und z.B. mit achsparallelen Schlitzen versehen ist.

10. Modul nach einem der Ansprüche 4 bis 9, dadurch **gekennzeichnet**, daß in der Kontaktzone (30) eine Lotschicht (60) oder Lotfüllung vorhanden ist, welche die ineinandersteckenden Enden des Elektroden-Ableitdrahtes (28) und des Mittenleiters (32) leitend verbindet.

11. Modul wenigstens nach Anspruch 6 und 10, dadurch **gekennzeichnet**, daß die Lotschicht (60) die Innenwandung des Sacklochs (34) in solcher Stärke (z.B. 0,1 mm) überzieht oder eine Lotfüllung im Sackloch (34) auf eine solche lichte Weite aufgebohrt ist, daß das freie Ende (36) des Ableitdrahtes (28) noch frei eintreten kann, sein Ringwulst (38) jedoch zu sattem Formschluß mit dem Lot (60) gelangt.

12. Modul nach wenigstens einem der Ansprüche 6 bis 11, dadurch **gekennzeichnet**, daß der Ringwulst (38) durch spanloses Drücken, Rollen o.dgl. am Ableitdraht (28) geformt ist.

13. Modul nach wenigstens einem der Ansprüche 1 bis 12, dadurch **gekennzeichnet**, daß der Ableitdraht (28) in einer Ringhalterung (66) gefaßt ist, bevorzugt unter Verklebung mittels Epoxidharz.

14. Modul nach Anspruch 13, dadurch **gekennzeichnet**, daß die Ringhalterung (66) einstückig mit dem Fuß (18) der Elektrode (16) ist.

15. Modul nach wenigstens einem der Ansprüche 1 bis 14, dadurch **gekennzeichnet**, daß der im Elektrodenfuß (18) gehalterte Ableitdraht (28) einen Stützteil (50) durchsetzt, an dessen Unterseite die Sensorfläche (48) angeordnet ist.

16. Modul nach wenigstens einem der Ansprüche 1 bis 15, dadurch **gekennzeichnet**, daß der Ableitdraht (28) mit seinem unteren Teil einen mit einem Bund (46) versehenen Abschnitt des Elektrodenfußes (18) durchsetzt, welcher mittels O-Ringen (44, 62) gegenüber dem Kopf (20) sowie dem Gehäuse (12) abgedichtet ist.

17. Modul nach Anspruch 16, dadurch **gekennzeichnet**, daß der Kopf (20) an seinem unteren Ende eine Schulter (40) aufweist oder bildet, die an einer Stufe (42) im Gehäuse (12) einen Anschlag findet.

18. Modul nach einem der Ansprüche 15 bis 17, **gekennzeichnet** durch solche Bemessung des Bundes (46), daß dieser im Gehäuse (12) satt passend geführt und ein Herausdrücken des Stützteils (50) bzw. der Sensorfläche (48) in den Meßkanal (14) ausgeschlossen ist.

19. Modul nach einem der Ansprüche 15 bis 18, dadurch **gekennzeichnet**, daß der Sensorfläche (48) im Meßkanal (14) eine Kuppe (54) gegenübersteht.

20. Modul nach Anspruch 19, dadurch **gekennzeichnet**, daß die Kuppe (54) von einem in das Gehäuse (12) quer zu dem Meßkanal (14) eingepaßten Propfen (56) gebildet ist, namentlich einem Rund- oder Ovalstab, einem Prismenkörper o.dgl.

Fig. 1

Fig. 2

Fig. 3

Europäisches
Patentamt

**EUROPÄISCHER RECHERCHENBERICHT**

Nummer der Anmeldung

EP 91 10 8406

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 414 032 (CONDUCTA)<br>* Zusammenfassung; Ansprüche; Figur 1 *<br>--- | 1-3,17 | G 01 N 27/28 |
| X | EP-A-0 303 979 (PFEIFER)<br>* Zusammenfassung; Spalte 4, Zeilen 23-30; Ansprüche 1-5; Figur 1 *<br>--- | 1-3,17 | |
| A | EP-A-0 017 236 (KERNFORSCHUNGSANLAGE JÜLICH)<br>* Zusammenfassung; Seite 4, Zeilen 17-26; Ansprüche 1-6; Figur 1 *<br>--- | 1-3,13 | |
| A | EP-A-0 150 864 (SUMITOMO)<br>* Zusammenfassung; Figur 2 *<br>--- | 1 | |
| A | GB-A-2 054 869 (FRESENIUS)<br>* Zusammenfassung; Figur 1 *<br>----- | 1,19 | |

|  | RECHERCHIERTE SACHGEBIETE (Int. Cl.5) |
|---|---|
|  | G 01 N |

Der vorliegende Recherchenbericht wurde für alle Patentansprüche erstellt

| Recherchenort | Abschlußdatum der Recherche | Prüfer |
|---|---|---|
| DEN HAAG | 07-10-1991 | KEMPF G.V. |

EPO FORM 1503 03.82 (P0403)